# EUROPEAN PATENT APPLICATION

(11) **EP 4 388 910 A1**
(43) Date of publication of application: **26.06.2024**
(21) Application number: 23218886.2
(22) Date of filing: 20.12.2023
(51) Int. Cl.: A41D 20/00, A42B 3/10, A42C 5/02, A61F 9/02

(54) **HELMET AND GOGGLES WITH BROW PAD**

(30) Priority: 20.12.2022 US 202263434090 P
(71) Applicant: Bell Sports, Inc., Scotts Valley, CA 95066-3438 (US)
(72) Inventor: PENNER, Benjamin, Santa Cruz, 95062 (US); KLOTZ, Brett, Salt Lake City, 84109 (US); MARTING, Gregory, Auburn, 23360 (US); TOLLETTE, Garrett, Santa Cruz, 95065 (US)
(74) Representative: Reed Smith LLP

(57) **Abstract**

A brow pad is provided. The brow pad can block and/or re-direct sweat away from the eyes of a user. The brow pad can include a fabric band configured to contact a forehead of the user, an enclosed channel disposed within the fabric band and extending from a first location proximate to a first end of the fabric band to a second location proximate to a second end of the fabric band opposite the first end, and a drainage strip disposed within the enclosed channel.

## Description

### CROSS-REFERENCE TO RELATED APPLICATION

This application claims the benefit of U.S. Provisional Application No. 63/434,090, filed Dec. 20, 2022, the disclosure of which is incorporated by reference in its entirety.

### BACKGROUND

Sweat management is a critical aspect of helmet and goggles performance that involves managing the sweat from the head of a user (e.g., a wearer) of the helmet or goggles during use. Users are often participating in an activity that requires physical exertion, causing perspiration. Examples of activities include, but are not limited to, skiing, snowboarding, bicycling, rollerblading, rock climbing, skate boarding, and motorcycling. If not properly managed, sweat can drip into the eyes, glasses, and goggles of the user, blurring vision of the user and increasing the risk of a crash and injury.

Accordingly, helmets designed to prevent the dripping of sweat into the eyes, glasses, and goggles of the user include features that can absorb or re-direct the sweat away from the eyes of the user. For example, known helmets can include a foam padding designed to absorb sweat of the user to prevent dripping and obfuscation of the user's vision. However, this padding can become saturated and nevertheless result in sweat dripping into the eyes of the user.

Accordingly, there is a need for improvements in sweat management in helmets.

### SUMMARY

Embodiments provide a brow pad configured to drain sweat away from eyes of a user. The brow pad can include a fabric band comprising an inner surface configured to contact a forehead of the user. The brow pad can also include an enclosed channel disposed within the fabric band and extending from a first location proximate to a first end of the fabric band to a second location proximate to a second end of the fabric band opposite the first end. The brow pad can also include a drainage strip disposed within the enclosed channel, the drainage strip comprising a hydrophobic material.

In some embodiments, the drainage strip can be raised relative to the first end and the second end of the drainage strip when the fabric band is applied to the forehead of the user. The drainage strip can be configured to route sweat from a center of the enclosed channel towards the first location and the second location.

In some embodiments, the enclosed channel can include a foam material at least partially surrounding the drainage strip.

In some embodiments, the hydrophobic material can include at least one of silicone, rubber, or plastic.

In some embodiments, the drainage strip can have a D-shaped cross-section.

In some embodiments, the drainage strip can have a U-shaped cross-section.

In some embodiments, the drainage strip can have a circular cross-section.

In some embodiments, the drainage strip can include a plurality of ridges.

In some embodiments, the drainage strip can be within about 20 millimeters of the bottom of the fabric band.

In some embodiments, the drainage strip can extend at least about 140 millimeters.

In some embodiments, the brow pad can also include one or more ventilation openings.

Embodiments provide a helmet including a brow pad configured to drain sweat away from eyes of a user. The helmet can include a helmet body comprising an outer surface and an inner surface opposite the outer surface. The helmet can also include an outer surface and an inner surface opposite the outer surface. The helmet can also include a fabric band disposed on the inner surface and configured to contact a forehead of the user. The helmet can also include an enclosed channel disposed within the fabric band and extending from a first location proximate to a first end of the fabric band to a second location proximate to a second end of the fabric band opposite the first end. The helmet can also include a drainage strip disposed within the enclosed channel, the drainage strip comprising a hydrophobic material.

In some embodiments, the drainage strip can be raised relative to the first end and the second end of the drainage strip when the fabric band is applied to the forehead of the user. The drainage strip can be configured to route sweat from a center of the enclosed channel towards the first location and the second location.

In some embodiments, the enclosed channel can include a foam material at least partially surrounding the drainage strip.

In some embodiments, the hydrophobic material can include at least one of silicone, rubber, or plastic.

In some embodiments, the drainage strip can have a D-shaped cross-section.

In some embodiments, the drainage strip can have a U-shaped cross-section.

In some embodiments, the drainage strip can have a circular cross-section.

In some embodiments, the drainage strip can include a plurality of ridges.

In some embodiments, the drainage strip can be within about 20 millimeters of the bottom of the fabric band.

In some embodiments, the drainage strip can extend at least about 140 millimeters.

In some embodiments, the brow pad can also include one or more ventilation openings.

Embodiments provide goggles configured to drain sweat away from eyes of a user. The goggles can include a frame. The goggles can also include a lens disposed within the frame. The goggles can also include an inner padding attached to an inner surface of the frame, the inner padding including a brow pad with an enclosed channel extending from a first location to a second location. The goggles can also include a drainage strip disposed within the enclosed channel, the drainage strip including a hydrophobic material. A center of the drainage strip can be raised relative to both ends of the drainage strip when the goggles are worn by the user. The drainage strip can be configured to route sweat from a center of the enclosed channel towards the first location and the second location.

In some embodiments, the enclosed channel can include a foam material at least partially surrounding the drainage strip.

In some embodiments, the hydrophobic material can include at least one of silicone, rubber, or plastic.

In some embodiments, the brow pad can include a first foam layer; a fabric band configured to contact a face of the user; and a second foam layer between the first foam layer and the fabric band. The enclosed channel can be defined within the second foam layer.

In some embodiments, the drainage strip can have a D-shaped cross-section.

In some embodiments, the drainage strip can have a U-shaped cross-section.

In some embodiments, the drainage strip can have a circular cross-section.

In some embodiments, the drainage strip can include a plurality of ridges.

In some embodiments, the drainage strip can be within about 20 millimeters of the bottom of the fabric band.

In some embodiments, the drainage strip can extend at least about 140 millimeters.

In some embodiments, the brow pad can also include one or more ventilation openings.

### BRIEF DESCRIPTION OF THE DRAWINGS

FIG. 1 illustrates a helmet having a sweat guiding brow pad according to exemplary embodiments.
FIG. 2 illustrates a sweat guiding brow pad according to exemplary embodiments.
FIG. 3 illustrates an enlarged view of the brow pad of FIG. 2 along a cross-section A-A according to exemplary embodiments.
FIG. 4 illustrates a brow pad according to exemplary embodiments.
FIG. 5 illustrates an enlarged view of the brow pad of FIG. 4 along a cross-section A-A according to exemplary embodiments.
FIGS. 6A-6H illustrate cross-sections of a drainage strip of a brow pad according to exemplary embodiments.
FIGS. 7A-7C illustrate a drainage strip of a goggle according to an exemplary embodiment.

### DETAILED DESCRIPTION

While structures are described herein by way of examples and embodiments, those skilled in the art recognize that the helmet with brow pad is not limited to the embodiments or drawings described. It should be understood that the drawings and description are not intended to be limited to the particular form disclosed. Rather, the intention is to cover all modifications, equivalents, and alternatives falling within the spirit and scope of the appended claims. Any headings used herein are for organizational purposes only and are not meant to limit the scope of the description or the claims. As used herein, the word "can" is used in a permissive sense (e.g., meaning being able to to) rather than the mandatory sense (e.g., meaning must). Similarly, the words "include," "including," and "includes" mean including, but not limited to.

This disclosure, its aspects, and implementations are not limited to the specific material types, components, methods, or other examples disclosed herein. Many additional material types, components, methods, and procedures are contemplated for use with particular implementations from this disclosure. Accordingly, for example, although particular implementations are disclosed, such implementations and implementing components can comprise any components, models, types, materials, versions, quantities, and/or the like as is known in the art for such systems and implementing components, consistent with the intended operation.

The word "exemplary," "example," or various forms thereof are used herein to mean serving as an example, instance, or illustration. Any aspect or design described herein as "exemplary" or as an "example" is not necessarily to be construed as preferred or advantageous over other aspects or designs. Furthermore, examples are provided solely for purposes of clarity and understanding and are not meant to limit or restrict the disclosed subject matter or relevant portions of this disclosure in any manner. It is to be appreciated that a myriad of additional or alternate examples of varying scope could have been presented, but have been omitted for purposes of brevity.

While this disclosure includes a number of implementations in many different forms, there are, shown in the drawings and will herein be described in detail, particular implementations with the understanding that the present disclosure is to be considered as an exemplification of the principles of the disclosed methods and systems, and is not intended to limit the broad aspect of the disclosed concepts to the implementations illustrated.

The present application relates to a brow pad that can be disposed on a helmet and configured to re-direct sweat away from and/or blocking from eyes of a user of the helmet. The brow pad can include a drainage strip that, when pressed against a forehead of the user, is raised at a central point relative to its ends, thereby blocking and/or draining the sweat along the drainage strip and away from the eyes of the user.

Applicant has invented a novel brow pad for a helmet that re-directs sweat away from the eyes of the user. The helmet can be any type or style of helmet, such as a cycling helmet, a skiing helmet, a snowboarding helmet, a skateboarding helmet, a motorcycle helmet, etc. The brow pad can be any suitable shape to be installed on an inner surface of the helmet. A person of ordinary skill in the art will appreciate that the exemplary brow pads described in this disclosure, e.g., 200, 400, are merely illustrative and in no way limiting of the possible shape/design of the brow pad.

While the embodiments disclosed herein describe a brow pad for a helmet that re-directs sweat away from the eyes of the user, it will be understood by a person of ordinary skill in the art that the brow pad can re-direct any liquid dripping from the forehead of the user, such as water.

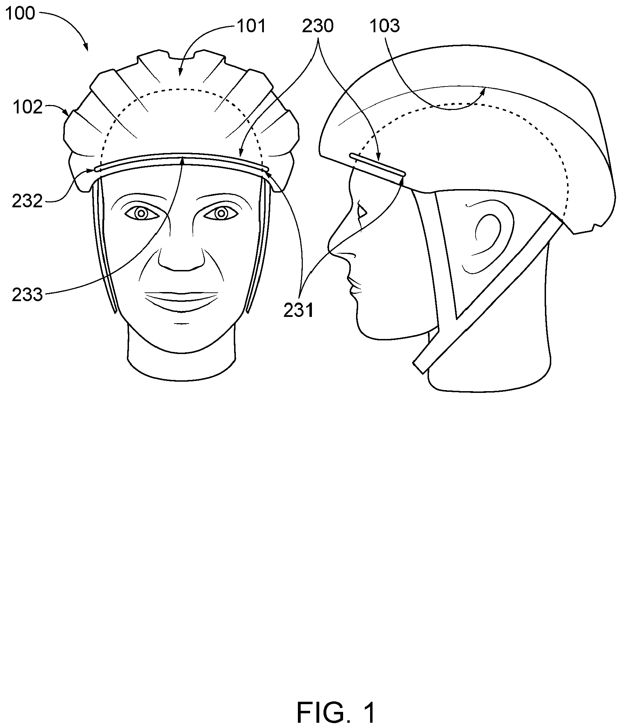

Referring to FIG. 1 according to an exemplary embodiment, a helmet 100 can include a helmet body 101 having an outer surface 102 and an inner surface 103 opposite outer surface 102. Helmet 100 is equipped with a drainage strip 230 configured to direct sweat from a forehead of a user away from eyes of the user and toward ends 231 and 232. When applied to the forehead of the user, drainage strip 230 can be raised at a central point 233 relative to ends 231 and 232 so that sweat that contacts drainage strip 230 is redirected along a length of drainage strip 230 and towards ends 231 and 232. Alternatively, drainage strip 230 may not be raised at a central point 233, in which case drainage strip 230 acts as a barrier, blocking sweat from the entering the eyes of the user.

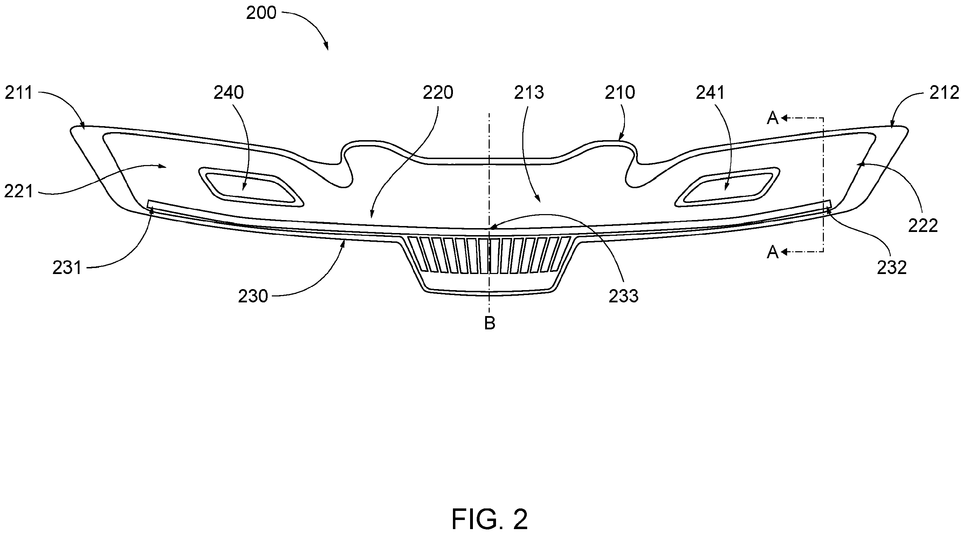

As seen in FIG. 2, exemplary embodiments of a brow pad 200 can include a fabric band 210 having a first end 211 and a second end 212 opposite first end 211 about an axis B, an enclosed channel 220 disposed within fabric band 210 and extending from a first location 221 to a second location 222, and a drainage strip 230 housed within enclosed channel 220.

In various embodiments, brow pad 200 can further include one or more ventilation openings, e.g., 240, 241. Ventilation openings can permit the flow of air through the brow bad to improve breathability.

In various embodiments, first location 221 can be proximate to first end 211 and second location 222 can be proximate to second end 212.

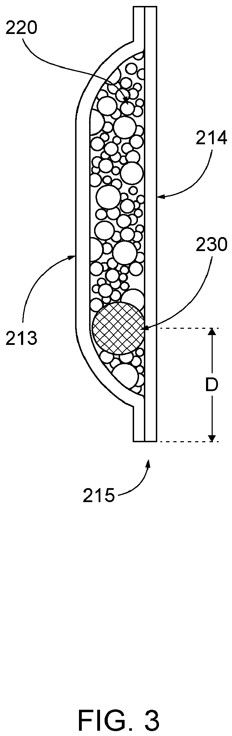

In various embodiments, enclosed channel 220 can be filled with a foam material that at least partially surrounds drainage strip 230, as illustrated in FIG. 3 according to an exemplary embodiment. In such embodiments, the foam material can fill an interior side of enclosed channel 220 but not an exterior side of enclosed channel 220.

Drainage strip 230 can extend a length from an end 231 and an end 232 located opposite end 231. In various embodiments, end 231 can be proximate to first location 221 and end 232 can be proximate to second location 222. In such embodiments, the length of drainage strip 230 is approximately equal to a length of enclosed channel 220.

In general, the length of drainage strip 230 can be any length sufficient to redirect sweat away from the eyes of the user in order to prevent the sweat from dripping into the eyes of the user. In various embodiments, the length of drainage strip 230 can be at least about 140 millimeters (mm), such as about 145 mm, such as about 150 mm, such as about 160 mm.

In various embodiments, drainage strip 230 can made of a hydrophobic material that repels sweat from the eyes of the user along the length of drainage strip 230 and towards ends 231 and 232. The hydrophobic material can be, but is not limited to, silicone, rubber, or plastic. It will be appreciated that the drainage strip can be made of any material capable of directing sweat away from the eyes of the user, including a composite material that has hydrophobic characteristics.

As seen in FIG. 3, drainage strip 230 can be positioned within enclosed channel 220 a distance D from a bottom 215 of fabric band 210. In various embodiments, distance D is about 20 mm or less, such as about 18 mm, such as about 15 mm. In various embodiments, drainage strip 230 can have a circular cross-section.

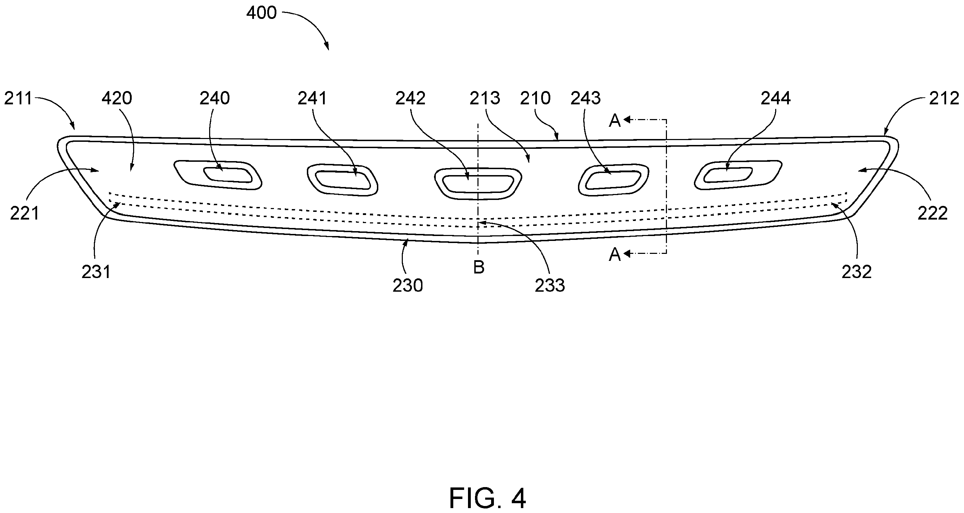

According to FIG. 4, alternative embodiments of a brow pad 400 can include a fabric band 210 having a first end 211 and a second end 212 opposite first end 211 about an axis B, an enclosed channel 420 disposed within fabric band 210 and extending from a first location 221 to a second location 222, and a drainage strip 230 including ends 231 and 232 housed within enclosed channel 220. Brow pad 400 can further include about five ventilation openings, e.g., 240, 241, 242, 243, 244.

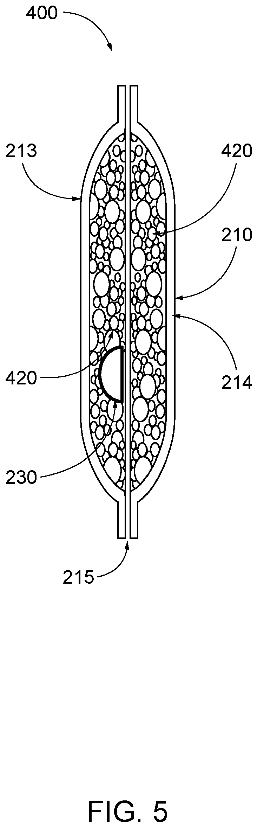

FIG. 5 illustrates a cross-section of brown pad 400 having a drainage strip 230 with a D-shaped cross-section according to an exemplary embodiment. While the rounded portion of the D-shaped cross-section is depicted as facing toward inner surface 213 of fabric band 210, it will be understood by a person of ordinary skill in the art that alternative embodiments exist in which the rounded portion faces away from inner surface 213 and the flat portion of the D-shaped cross-section faces toward inner surface 213.

As illustrated in FIG. 5, brow pad 400 includes an enclosed channel 420 filed with a foam material on both an interior side and an exterior side of fabric band 210, increasing the level of comfort of helmet 100 on the user when brow pad 400 is applied to the forehead of the user.

When applied to helmet 100, an inner surface 213 of fabric band 210 can contact a forehead of the user and an outer surface 214 can be configured to secure brow pad 200, 400 to inner surface 103 of helmet 100 by, e.g., Velcro or a snap-fit attachment. Drainage strip 230 can be raised at a central point 233, as seen in FIG. 1, relative to ends 231 and 232 when fabric band 210 is applied to the forehead of the user. When the user perspires, sweat from the forehead of the user is re-directed by drainage strip 230 to ends 231 and 232, thus preventing sweat from dripping into the eyes of the user. In various embodiments, central point 233 can coincide with axis B of fabric band 210. In other embodiments, central point 233 can be offset from axis B.

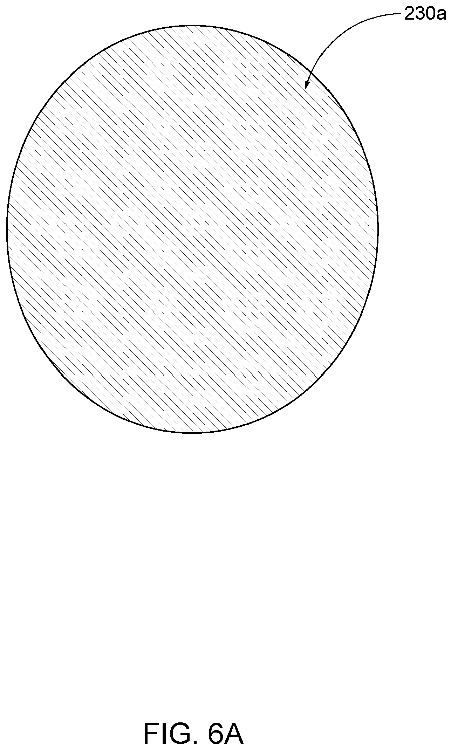

A cross-section of a drainage strip 230 can vary, as depicted in FIGS. 6A-F according to exemplary embodiments. In various embodiments, drainage strip 230 can have an elliptical cross-section, a U-shaped cross-section, a D-shaped cross-section, a B-shaped cross-section, an E-shaped cross-section, or an O-shaped cross-section.

As illustrated in FIG. 6A, drainage strip 230a can have an elliptical cross-section, such as an oval or a circle.

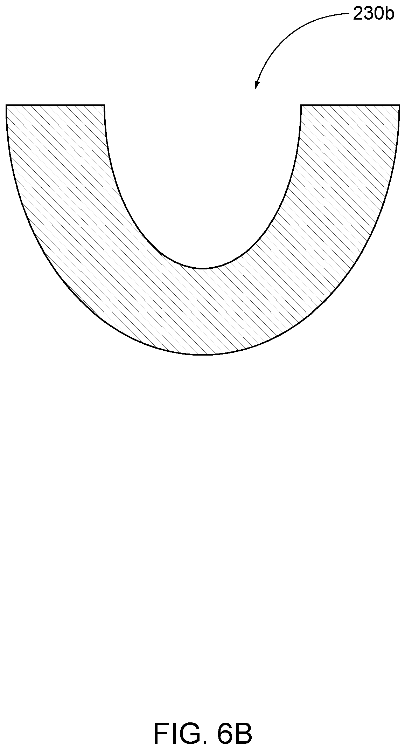

As illustrated in FIG. 6B, drainage strip 230b can have a U-shaped cross-section.

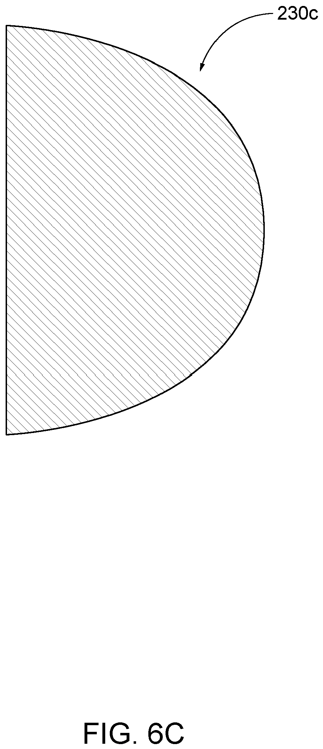

In FIG. 6C, drainage strip 230c can have a single ridge 233. In such embodiments, a cross-section of drainage strip 230c can be D-shaped.

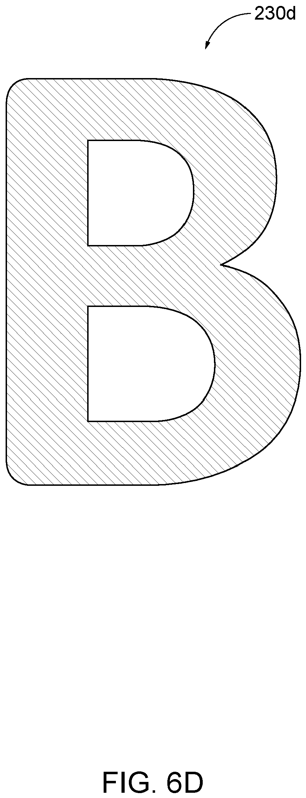

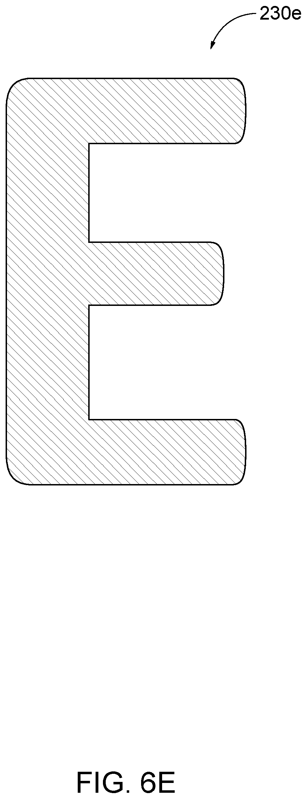

FIGS. 6D and 6E illustrates a drainage strip 230d, 230e can have a plurality of ridges. In FIG. 6D, drainage strip 230d can have two ridges. In such embodiments, a cross-section of drainage strip 230d can be B-shaped. In FIG. 6E, drainage strip 230e can have three ridges. In such embodiments, a cross-section of drainage strip 230e can be E-shaped.

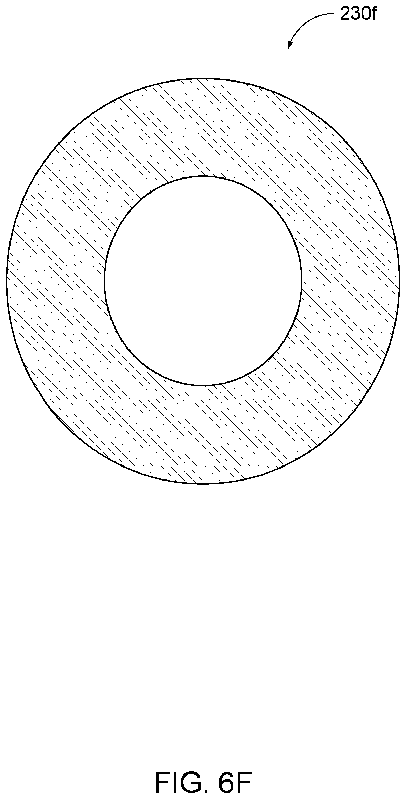

In FIG. 6F, a drainage strip 230f can have a hollow center. In such embodiments, a cross-section of drainage strip 230f can be O-shaped.

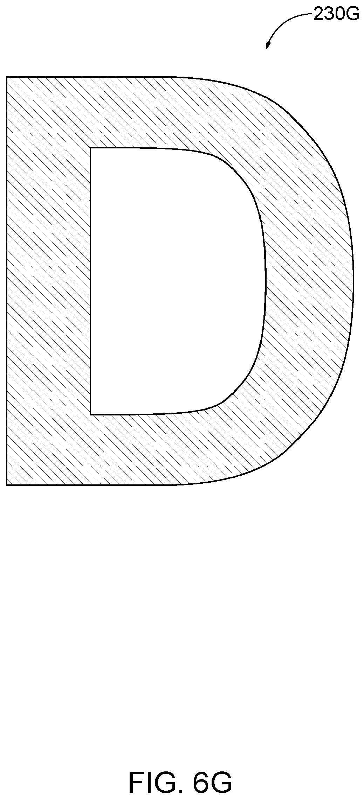

In FIG. 6G, a drainage strip 230g can be a variation of drainage strip 230c in which a cross-section of drainage strip 230g is a hollow D-shape.

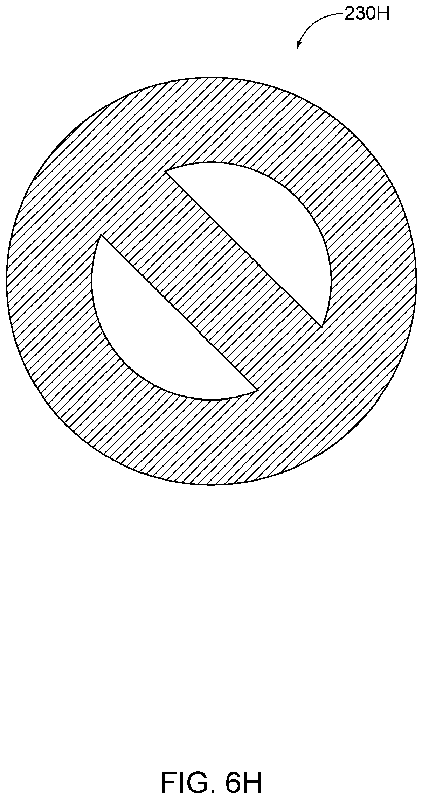

In FIG. 6H, a drainage strip 230h can be a variation of drainage strip 230b in which a cross-section of drainage strip 230h is an O-shape with a connection through a center.

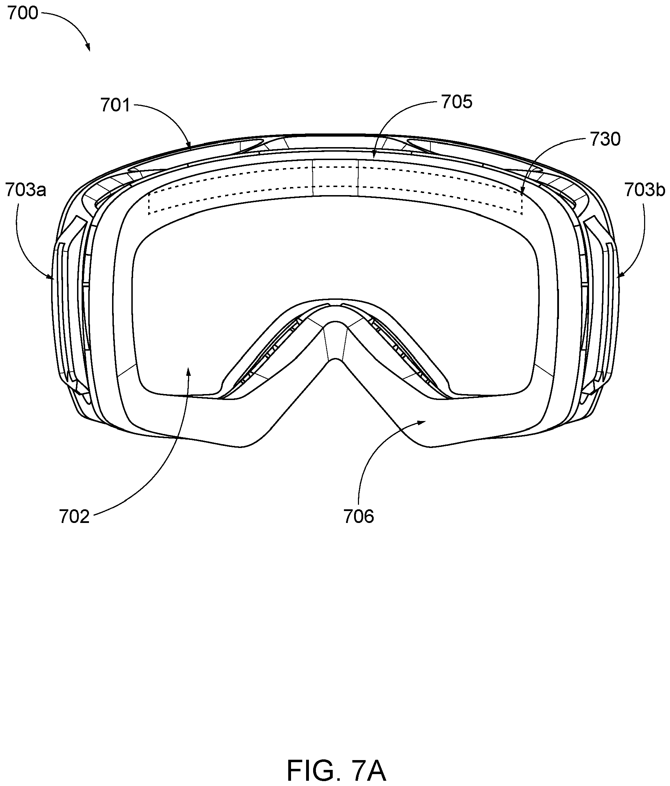

FIGS. 7A-7C illustrate an exemplary embodiment of a drainage strip implemented in a pair of goggles 700. Pair of goggles 700 can be any style of goggle, for example, used in skiing, snowboarding, bicycling, and the like. Pair of goggles 700 can include a frame 701, a lens 702, and a pair of attachment points 703a and 703b for attaching a strap (not illustrated) configured to secure pair of goggles 700 to the head and/or helmet of a user. A drainage strip 730 can be inlaid within an enclosed channel 704 of a brow pad 705 an inner padding 706 of pair of goggles 700. Inner padding 706 can be shaped to fit along an inner perimeter surface of frame 701. When in use, an inner surface of inner padding 706 rests around the eyes of a user allowing the user to see through lens 702.

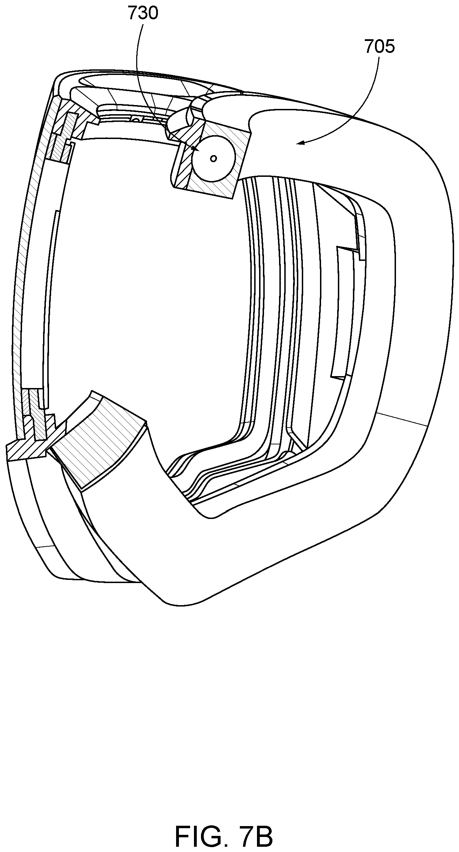

FIG. 7B illustrates a cross-section of goggles 700 in which drainage strip 730 is inlaid within brow pad 705. Inner padding 706 can be made of a foam material and include a single layer of foam or more than one layer of foam. Inner padding 706 can further include a fabric band that contacts the face of the user when in use.

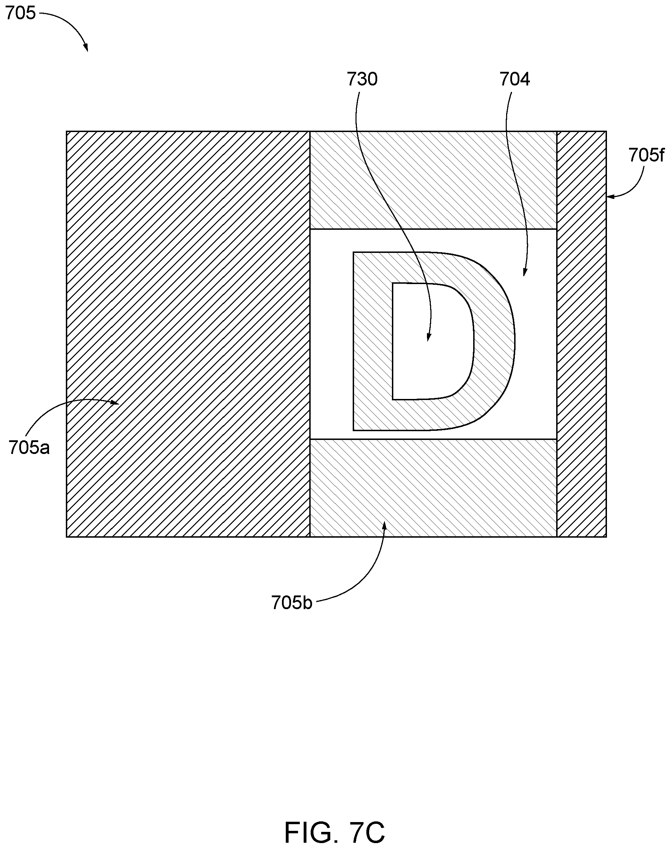

FIG. 7C illustrates an enlarged view of a cross-section of brow pad 705. Brow pad 705 can include a first layer of foam 705a, a second layer of foam 705b, and fabric band 705f. Drainage strip 730 can be housed within enclosed channel 704 defined within second foam layer 705b of brow pad portion 705. Alternatively, enclosed channel 704 can be defined in first layer of foam 705a. While illustrated with two layers of foam, a person of ordinary skill will understand that brow pad 705 can alternatively include a single layer of foam or more than two layers of foam. Drainage strip 730 as illustrated has a can be drainage strip 230h having a hollow D-shaped cross-section, as illustrated in FIG. 6G. However, it will be appreciated by one of ordinary skill in the art that any variation of drainage strip can be used, e.g., drainage stipes 230a-230h, without departing from the scope of this disclosed.

When in use, brow pad 705 presses against the face above the eyes of the user, such that drainage strip 730 can block sweat and/or other liquid dripping from the forehead of a user. Drainage strip 730 can be raised at a point at or near a central point of the brow pad or channel in order to re-direct sweat away from the eyes of the user along a length of drainage strip 730. Alternatively, the drainage strip 730 can have a straight profile without any rise at the central point and can function to block sweat and/or other liquid from entering the eyes of the user.

It will be understood that helmet with brow pad implementations are not limited to the specific components disclosed herein, as virtually any components consistent with the intended operation of the various helmet with brow pad implementations can be utilized. Accordingly, for example, it should be understood that, while the drawings and accompanying text show and describe particular helmet with brow pad implementations, any such implementation can comprise any shape, size, style, type, model, version, class, grade, measurement, concentration, material, weight, quantity, and/or the like consistent with the intended operation of helmet with brow pad implementations.

The concepts disclosed herein are not limited to the specific helmet with brow pad implementations shown herein. For example, it is specifically contemplated that the components included in particular helmet with brow pad implementations can be formed of any of many different types of materials or combinations that can readily be formed into shaped objects and that are consistent with the intended operation of the helmet with brow pad implementations. For example, the components can be formed of: silicones and/or other like materials; rubbers (synthetic and/or natural) and/or other like materials; elastomers and/or other like materials; polymers and/or other like materials; plastics and/or other like materials; composites and/or other like materials; and/or any combination of the foregoing.

Furthermore, helmet with brow pad implementations can be manufactured separately and then assembled together, or any or all of the components can be manufactured simultaneously and integrally joined with one another. Manufacture of these components separately or simultaneously, as understood by those of ordinary skill in the art, can involve extrusion, pultrusion, vacuum forming, injection molding, blow molding, resin transfer molding, and/or the like. If any of the components are manufactured separately, they can then be coupled or removably coupled with one another in any manner, such as with adhesive, a plastic weld, a fastener, any combination thereof, and/or the like for example, depending on, among other considerations, the particular material(s) forming the components.

In places where the description above refers to particular helmet with brow pad implementations, it should be readily apparent that a number of modifications can be made without departing from the spirit thereof and that these implementations can be applied to other implementations disclosed or undisclosed. The presently disclosed helmet with brow pad implementations are, therefore, to be considered in all respects as illustrative and not restrictive.

Having described and illustrated the principles of our invention with reference to the described embodiment, it will be recognized that the described embodiment can be modified in arrangement and detail without departing from such principles. It should be understood that the programs, processes, or methods described herein are not related or limited to any particular type of computing environment, unless indicated otherwise. Elements of the described embodiment shown in software can be implemented in hardware and vice versa.

In view of the many possible embodiments to which the principles of our invention can be applied, we claim as our invention all such embodiments as can come within the scope and spirit of the following claims and equivalents thereto.

## Claims

1. A brow pad (200, 400, 705) configured to drain sweat away from eyes of a user, comprising:
a fabric band (210) comprising an inner surface configured to contact a forehead of the user;
an enclosed channel (220) disposed within the fabric band and extending from a first location (221) proximate to a first end (211) of the fabric band to a second location (222) proximate to a second end (212) of the fabric band opposite the first end; and
a drainage strip (230) disposed within the enclosed channel, the drainage strip comprising a hydrophobic material.

2. The brow pad (200, 400, 705) of claim 1, wherein a central point (233) of the drainage strip is raised relative to a first end (231) of the drainage strip, located proximate to the first location and a second end (232) of the drainage strip, located proximate to the second location, when the fabric band is applied to the forehead of the user, and
wherein the drainage strip is configured to route sweat from a center of the enclosed channel towards the first location and the second location.

3. The brow pad (200, 400, 705) of claim 1 or 2, wherein the enclosed channel comprises a foam material at least partially surrounding the drainage strip.

4. The brow pad (200, 400, 705) of any of claims 1 to 3, wherein the drainage strip has a D-shaped cross-section.

5. The brow pad (200, 400, 705) of any of claims 1 to 3, wherein the drainage strip has a U-shaped cross-section.

6. The brow pad (200, 400, 705) of any of claims 1 to 3, wherein the drainage strip has a circular cross-section.

7. The brow pad (200, 400, 705) of any of claims 1 to 3, wherein the drainage strip comprises a plurality of ridges.

8. The brow pad (200, 400, 705) of any of claims 1 to 7, wherein the hydrophobic material comprises one of: silicone, rubber, or plastic.

9. The brow pad of any of claims 1 to 8, wherein the drainage strip is within about 20 millimeters of a bottom (215) of the fabric band, the bottom of the fabric band being disposed towards the user's eyes when the fabric band is in contact with the user's forehead.

10. The brow pad (200, 400, 705) of any of claims 1 to 9, wherein the drainage strip has a length of at least about 140 millimeters as measured along the enclosed channel in a direction from the first location to the second location.

11. The brow pad of any of claims 1 to 10, wherein:
the brow pad comprises a first foam layer (705a) and a second foam layer (705b) between the first foam layer and the fabric band, and
the enclosed channel is defined within the second foam layer.

12. A helmet (100) configured to drain sweat away from eyes of a user, the helmet comprising:
a helmet body (101) comprising an outer surface (102) and an inner surface (103) opposite the outer surface;
a brow pad (200, 400, 705) according to any of claims 1 to 11, the fabric band of the brow pad being disposed on the inner surface of the helmet body and configured to contact a forehead of the user.

13. The helmet of claim 12, further comprising one or more ventilation openings (240, 241).

14. Goggles (700) configured to drain sweat away from eyes of a user, the goggles comprising:
a frame (701);
a lens (702) disposed within the frame;
an inner padding (706) attached to an inner surface of the frame, the inner padding comprising a brow pad (200, 400, 705) according to any of claims 1 to 11.
